# EUROPEAN PATENT APPLICATION

(11) **EP 2 502 931 A1**
(43) Date of publication of application: **26.09.2012**
(21) Application number: 10831473.3
(22) Date of filing: 08.11.2010
(51) Int. Cl.: C07J 71/00, A61K 8/63, A61Q 19/02

(54) **INHIBITOR OF DOPA OXIDASE ACTIVITY AND SKIN LIGHTENING AGENT**

(30) Priority: 19.11.2009 JP 2009263588
(71) Applicant: Kao Corporation, Chuo-Ku Tokyo 103-8210 (JP)
(72) Inventor: KAWASAKI, Akiko, Haga-gun Tochigi 321-3497 (JP); SUGIYAMA, Mitsuru, Haga-gun Tochigi 321-3497 (JP); AMANO, Yasuko, Tokyo 131-8501 (JP)
(74) Representative: Vossius & Partner
(86) International application number: PCT/JP2010/069827
(87) International publication number: WO 2011/062078

(57) **Abstract**

An inhibitor of dopa oxidase activity containing a compound represented by the following Formula (1) as an active ingredient, and a skin-lightening agent containing the compound as an active ingredient: 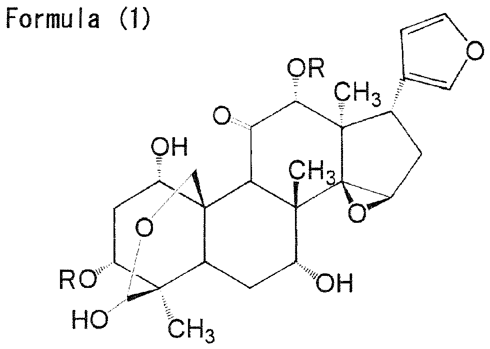 wherein R represents an acyl group having 1 to 5 carbon atom(s).

## Description

### TECHNICAL FIELD

The present invention relates to an inhibitor of dopa oxidase activity and a skin-lightening agent.

### BACKGROUND ART

Pigmentation after suntan, and blotches and freckles are generally considered to be generated as a result of an activation of pigment cells (melanocytes) presented in a skin followed by an increase in a melanin production, due to a stimulation of the skin by an ultraviolet exposure, abnormal secretion of hormones, genetic factors, or the like. In an organism, a pigment melanin is biosynthesized from tyrosine, which is a precursor of the melanin, at the melanosome in the pigment cells (melanocytes) by an action of an enzyme tyrosinase. It has been reported that when a mutation is occurred in the tyrosinase, which is an enzyme involved in a melanin biosynthesis, a formation of a melanin pigment in a skin and a hair becomes anomalous (for example, see non-patent literature 1).

Tyrosinase, which plays important role in the melanin biosynthesis, has been considered to be useful as a target of skin lightening materials, and studies of skin-lightening materials have been made with paying attention to tyrosinase hitherto. Tyrosinase specifically has tyrosine hydroxylase activity, dopa oxidase activity, and DHI activity, and catalyzes a reaction of a melanin synthesis which uses tyrosine and dopa as precursors. Therefore, when investigating the tyrosinase enzymatic activity, for example, the dopa oxidase activity or the like can be used as an index. In fact, the dopa oxidase activity has been used as an index, when evaluating a suppressing material(s) of a melanin production which has an inhibitory effect of a tyrosinase enzymatic activity (for example, see non-patent literature 2). According to this technique, materials which enable to suppress a production of melanin which is a final biosynthesis product can be evaluated, by suppressing dopa oxidase activity in the melanocyte.

As such, a research has been hitherto made on materials capable of suppressing melanin production and materials capable of reducing produced melanin by inhibiting an activity of tyrosinase, and the like, and use of these materials as a skin-lightening component has been studied. For example, ascorbic acid, arbutin, kojic acid, glutathione and the like have been reported to have the above function (for example, see non-patent literature 3).

### PRIOR ART LITERATURE

### NON-PATENT LITERATURES

Non-Patent Literature 1: King RA. Oetting WS. Hearing VJ. In Metabolic bases of inherited disease (Scriver CR. Beaudet AL. Sly WS. Valle D., eds.), McGraw-Hill, New York, 4353-4392, 1995
Non-Patent Literature 2: Wrathall JR. et al., JCB 1973 57: 406-423
Non-Patent Literature 3: Skin Whitening Strategy (Nankodo Co., Ltd.) Part IV., Pharmacology and Clinical Practice for Skin-Lightening Agents, p95-115

### SUMMARY OF INVENTION

The present invention is contemplated for providing an inhibitor of dopa oxidase activity, which is capable of effectively inhibiting dopa oxidase activity. Further, the present invention is contemplated for providing a skin-lightening agent which suppresses melanin production by inhibiting dopa oxidase activity.

In view of the purposes described above, the inventors of the present invention have made extensive studies to explore new materials having skin-lightening action. As a result, they have found out that a compound represented by the following Formula (1) has an inhibitory action on dopa oxidase activity, and obtained a finding that this compound is useful as a novel skin-lightening component. The present invention has been completed based on these findings.

The present invention provides the following means:
(1) An inhibitor of dopa oxidase activity, containing a compound represented by the following Formula (1) as an active ingredient.

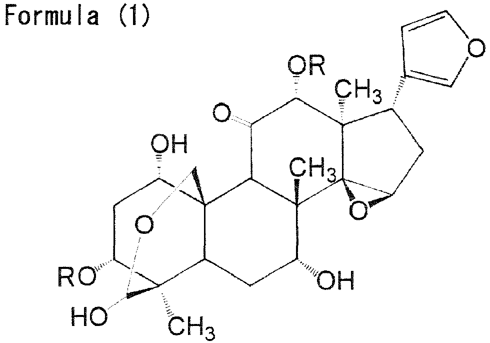

wherein R represents an acyl group having 1 to 5 carbon atom(s).
(2) A skin-lightening agent, containing the compound represented by the above Formula (1) as an active ingredient.
(3) The compound represented by the above Formula (1) for use in the suppression of dopa oxidase activity.
(4) The compound represented by the above Formula (1) for use in the skin-lightening.
(5) Use of the compound represented by the above Formula (1) for the preparation of a medicament having an effect of suppressing dopa oxidase activity.
(6) Use of the compound represented by the above Formula (1) for the preparation of a medicament having an effect of skin-lightening.

The inhibitor of dopa oxidase activity and the skin-lightening agent of the present invention contain, as an active ingredient, the compound represented by the above Formula (1) which has an excellent activity to suppress dopa oxidase activity. Therefore, the inhibitor of dopa oxidase activity and the skin-lightening agent of the present invention can suppress the dopa oxidase activity to thereby suppress the tyrosinase-induced melanin production, result in showing prophylactic or preventive effects on blotches and freckles or on pigmentation after suntan.

Other and further features and advantages of the present invention will appear more fully from the following description.

### MODE FOR CARRYING OUT INVENTION

Hereinafter, the present invention is described in detail.
The inhibitor of dopa oxidase activity and the skin-lightening agent of the present invention contain a compound represented by the following Formula (1), as an active ingredient.

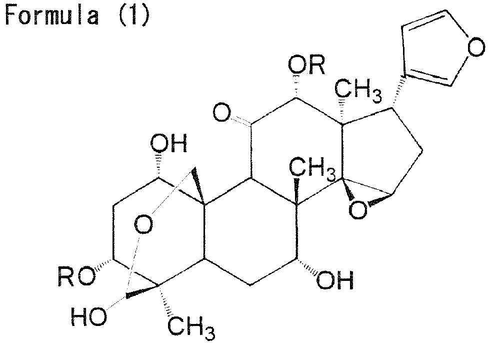

wherein R represents an acyl group having 1 to 5 carbon atom(s).
R is preferably an acyl group having 1 to 3 carbon atom(s), and more preferably an acyl group having two carbon atoms. Specific examples of R include a formyl group, an acetyl group, a propionyl group, a butyryl group, an isobutyryl group, a valeryl group and an isovaleryl group. Among them, a formyl group, an acetyl group, and a propionyl group are preferred, and an acetyl group is more preferred. A compound represented by the Formula (1) in which R is an acetyl group is toosendanin, which is a kind of terpene.
The compound represented by the Formula (1) may adopt tautomeric forms as shown below, and the compound represented by the Formula (1) of the present invention encompasses both of the tautomers.

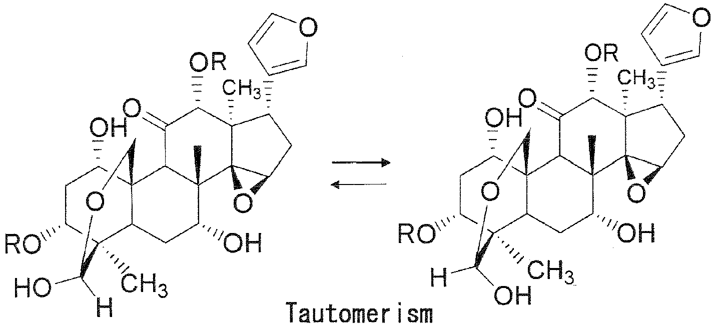

The method for producing the compound represented by the above Formula (1) used in the present invention is not particularly limited. A chemically synthesized compound may be used, and a compound extracted or purified from a natural product-derived material may also be used. Furthermore, a product that is commercially available as a reagent can also be used.
As the reagent, a product commercially available from Avachem Scientific LLC (USA) and the like can be used.

With regard to the method of obtaining the compound represented by the above Formula (1) from a natural product-derived material, for example, the compound can be isolated from plants such as *Melia toosendan* Sieb. et Zucc., *Melia azedarach*, and *Toona sinensis.*
For these plants, any and all parts (whole tree, whole grass, roots, rhizomes, trunks, branches, stems, leaves, barks, tree sap, tree resin, flowers, fruits, seeds and the like of the plant) can be used. Particularly, in the case of isolating the compound from *Melia toosendan* Sieb. et Zucc., it is preferable to use the barks, seeds or fruits of the plant. Further, an herbal medicine obtained by using the fruits of *Melia toosendan* Sieb. et Zucc. as an original plant, Senrenshi, can also be used. In the case of isolating the compound from *Melia azedarach,* it is preferable to use the barks of the plant. An herbal medicine obtained by using the barks of *Melia azedarach* as a original plant, Kurenpi, can also be used. In the case of isolating the compound from *Toona sinensis,* it is preferable to use the barks of the plant.
The compound represented by the Formula (1) can also be isolated by using these various plants or various parts in appropriate combination.

The method of isolating the compound represented by the above Formula (1) from these plants is not particularly limited, but an example may be a method in which the plant mentioned above is applied to the extraction using an appropriate solvent, and isolating the compound represented by the above Formula (1) from the obtained plant extract by using a technique such as chromatography.
The plant can be used directly or after drying and grinding, for the preparation of the plant extract. As a solvent that is used for the extraction, those conventionally used for the extraction of plant components, for example, water, petroleum ether, n-hexane, toluene, chloroform, ether, ethyl acetate, acetone, methanol, ethanol, propanol, butanol, ethylene glycol, propylene glycol, butylene glycol and mixed solutions thereof, can be used. Further, with regard to the extraction condition, typical extraction conditions can be employed. For example, the above-described plant may be dipped or heated under reflux for two hours to 60 days at 5 to 80°C.
Specific methods for extraction and isolation that can be used include the methods shown in the following Examples, but the present invention is not limited thereto.

As demonstrated in the Examples described below, the compound represented by the above Formula (1) effectively suppresses dopa oxidase activity. Therefore, the skin-lightening agent of the present invention which contains the compound can inhibit tyrosinase activity and shows a skin-lightening effect by suppressing the production of melanin in the skin. Furthermore, the compound represented by the above Formula (1) can also be used as an inhibitor of dopa oxidase activity or an inhibitor of tyrosinase activity.
JP-T-2006-514657 discloses that a composition containing Toosendanin is used in the prevention and improvement of wrinkles. It has also been known that Senrenshi and Kurenpi, which are herbal medicines, show an anthelmintic action. However, the fact that the compound represented by the above Formula (1) shows an action of effectively inhibiting dopa oxidase activity and is useful as a skin-lightening component is a finding newly obtained by the inventors of the present invention.
According to the present invention, the term "skin-lightening (action)" means an action of suppressing the production of melanin pigment and thereby returning a skin color to the original transparent tone without any extra melanin, or an action of preventing and/or suppressing darkening of a skin or pigmentation such as blotches and freckles.

According to the present invention, the compound represented by the above Formula (1) may be directly used as an inhibitor of dopa oxidase activity or a skin-lightening agent. Alternatively, the compound may also be used as a formulation prepared by adding thereto an appropriate liquid or solid excipient or extending agent such as titanium oxide, calcium carbonate, distilled water, lactose or starch. In this case, the amount of the compound represented by the above Formula (1) is not particularly limited, but it is preferable that the compound represented by the Formula (1) be contained in an amount of 0.00001 to 3% by mass, and more preferably 0.01 to 1% by mass.

Particularly, in the case of using the compound represented by the above Formula (1) as a skin-lightening agent, other skin-lightening component(s) may be used in addition to the compound. When the skin-lightening agent of the present invention is used as a composition together with other skin-lightening component(s), the amount of the compound represented by the above Formula (1) is not particularly limited, but it is preferable that the compound represented by the above Formula (1) be contained in an amount of 0.00001 to 3% by mass, and more preferably 0.01 to 1% by mass.

The inhibitor of dopa oxidase activity and the skin-lightening agent of the present invention can be used in the form of an external preparation for skin. The "external preparation for skin" means a formulation that is applied to the skin as a cosmetic material for skin, a drug for external use, a quasi-drug for external use, or the like. The dosage form can be in a wide variety of forms such as an aqueous solution system, a solubilized system, an emulsified system, a powder system, a gel system, an ointment system, a cream, water-oil biphasic system, and a water-oil-powder triphasic system. Examples of the dosage form include a face wash, a skin toner, an emulsion, a cream, a gel, an essence (serum), a facial pack, a facial mask, a foundation, an ointment, and a sheet-like product.
When used in the form of an external preparation for skin, components that are used in conventional external preparations for skin, for example, a surfactant, an oily material, a polymer compound, a preservative, efficacious ingredients other than the skin-lightening components mentioned above, a powder, an ultraviolet absorbent, a colorant, a fragrance, an emulsification stabilizer, and a pH adjusting agent can be appropriately incorporated into the external preparation, in addition to the compound represented by the above Formula (1). The dosage amount of the external preparation for skin containing the compound represented by the above Formula (1) may vary according to the content of the active ingredient, however, for example, in the case of a cream form or an ointment form, the dosage amount is preferably 0.1 to 5 µg per 1 square centimeter of a skin, and in the case of a liquid preparation, the dosage amount of use is preferably 0.1 to 10 µg per 1 square centimeter of a skin.

### EXAMPLES

Hereinafter, the present invention is described more in detail with reference to Examples, but the present invention is not limited thereto.

### Preparation Example

The fruits of *Melia toosendan* Sieb. et Zucc. (Senrenshi, manufactured by Shinwa Bussan Co., Ltd.) 800 g was extracted with 8 L of 50% ethanol at 20 to 35°C for 7 days, and the solvent was concentrated. Thus, 123.5 g of an extracted solid fraction was obtained. The obtained extracted solid fraction was fractionated based on dopa oxidase activity as an index. Liquid-liquid distribution was carried out using water and ethyl acetate, and the inhibitory activity was concentrated in 20.78 g of the ethyl acetate layer (yield 16.8%). The ethyl acetate layer was further fractionated by silica gel column chromatography, and thus Fraction (1) 7.49 g was obtained (yield 6.1%). This fraction was further fractionated using silica gel column chromatography, and thus Fraction (2) 2.78 g was obtained (yield 2.3%). Subsequently, this fraction was fractionated using LH-20 (Sephadex (trade mark) LH20, manufactured by GE Healthcare Inc.), and thus Fraction (3) 1.28 g was obtained (yield 1.0%). Fraction (3) 340 mg was fractionated by HPLC, and thus Fraction (4) 164.8 mg was obtained (yield 0.48%). Furthermore, 20 mg of Fraction (4) was fractionated by HPLC, and thus Fraction (5) 1.4 mg was obtained (yield 0.03%). This Fraction (5) was subjected to a structure analysis using NMR. In the structure analysis by NMR, toosendanin which is commercially available as a reagent (manufactured Avachem Scientific LLC) was used for comparison. The results of the structure analysis using NMR are shown in Table 1.
As a result, the active component isolated from *Melia toosendan* Sieb. et Zucc. was a compound having a structure shown in the following Table 1, and this compound was identified as toosendanin. in Table 1, the abbreviation Ac represents an acetyl group.

**Table 1**

| | 13C-NMR (ppm) | | 1H-NMR (ppm) | |
|---|---|---|---|---|
| | Isolated component | Toosendanin (reagent) | Isolated component | Toosendanin (reagent) |
| 1 | 70.7 | 70.7 | 4.24 | 4.24 |
| 2 | 37.2 | 37.2 | 1.8 | 1.8 |
| | | | 2.73 | 2.73 |
| 3 | 74.8 | 74.8 | 5.19 | 5.19 |
| 4 | 41.2 | 41.2 | - | - |
| 5 | 29.6 | 29.6 | 2.8 | 2.8 |
| 6 | 26.2 | 26.2 | 1.71 | 1.71 |
| | | | 2 | 2 |
| 7 | 70.8 | 70.9 | 3.57 | 3.57 |
| 8 | 43.9 | 43.9 | - | - |
| 9 | 50.1 | 50.1 | 4.7 | 4.7 |
| 10 | 42.9 | 42.9 | - | - |
| 11 | 209.1 | 209.1 | - | - |
| 12 | 79.7 | 79.7 | 5.33 | 5.33 |
| 13 | 46.9 | 46.9 | - | - |
| 14 | 73.6 | 73.6 | - | - |
| 15 | 60 | 60 | 3.81 | 3.8 |
| 16 | 34.8 | 34.8 | 2.02 | 2.01 |
| | | | 2.11 | 2.11 |
| 17 | 39.9 | 39.9 | 2.88 | 2.88 |
| 18 | 15.9 | 15.8 | 1.37 | 1.38 |
| 19 | 65.5 | 65.5 | 4.25 | 4.24 |
| | | | 4.31 | 4.31 |
| 20 | 124.3 | 124.3 | - | - |
| 21 | 142.1 | 142.1 | 7.2 | 7.2 |
| 22 | 113 | 113 | 6.16 | 6.16 |
| 23 | 143.7 | 143.7 | 7.4 | 7.4 |
| 28 | 20 | 20 | 0.84 | 0.84 |
| 29 | 97.2 | 97.2 | 4.83 | 4.83 |
| 30 | 23.1 | 23.2 | 1.12 | 1.12 |
| AcO | 21.4 | 21.4 | 2.07 | 2.07 |
| | 172.9 | 172.9 | - | - |
| | 20.9 | 20.9 | 1.95 | 1.95 |
| | 172.2 | 172.2 | - | - |

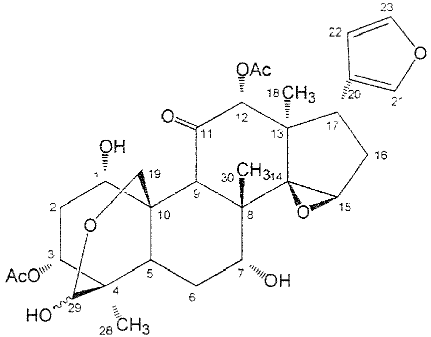

### Test Example Measurement of dopa oxidase activity

100 µL of human neonatal foreskin-derived melanocytes were inoculated on each well of a 96-well plate to be a cell density of 1×10⁴ cells/well. The medium used therein was Medium 254 to which Human Melanocyte Growth Supplement excluding PMA (all manufactured by Cascade Biologics Inc.) was added.
After culturing the cells for 24 hours, endothelin-1 (ET-1) which is a melanocyte activator, stem cell growth factor (SCF), α-melanocyte stimulating hormone (α-MSH), histamine and prostaglandin E₂ (PGE₂) were mixed and added to the culture to obtain a final concentration in medium of 10×10⁻⁷ mol/m³ respectively.

As a sample, toosendanin which is the compound isolated in Preparation Example was added thereto at a final concentration of 100 nM. Furthermore, cultures containing kojic acid as a reference sample at final concentrations of 10 µM and 1 mM were prepared. Kojic acid is a known skin-lightening component having high tyrosinase inhibitory activity.
Culturing was performed for 3 days at a final amount of medium of 200 µL/well under the conditions of 37°C and 5% CO₂.

The following additives had also been added to the medium.

| | |
|---|---|
| bFGF (basic fibroblast growth factor) | 3 ng/mL |
| BPE (Bovine Pituitary Extracting liquid) | 0.2 volume% |
| FBS(Fetal bovine serum) | 0.5 volume% |
| Hydrocortisone | 5 × 10⁻⁴ mol/m³ |
| Insulin | 5 µg/mL |
| Transferrin | 5 µg/mL |
| Heparin | 5 µg/mL |

After completing culture, the melanocytes were washed with Phosphate-buffered saline (PBS) without Ca²⁺ and Mg²⁺, and 20 µL/well of an extraction buffer (0.1 M Tris-HCl (pH 7.2), 1% Nonidet P-40, 0.01% SDS, 100 µM phenylmethylsulfonyl fluoride (PMSF) and 1 µg/ml aprotinin) and 20 µL/well of an assay buffer (100 mM Sodium phosphate-buffer (pH 7.1) containing 4% dimethylformamide) were added thereto, thereby lysing cells for 3 hours at 4°C, and the dopa oxidase activity was measured. The measurement of the dopa oxidase activity was carried out as follows, with reference to the MBTH method (see, for example, Winder A.J., Harris H., Eur. J. Biochem., 198, 317-326, 1991).

To each well containing the lysed cell solution, 80 µL/well of the assay buffer, 60 µL of a 20.7 mM 3-methyl-2-benzothiazolinonehydrazone (MBTH) solution, and 40 µL of a 5 mM L-dopa (L-dihydroxyphenylalanine) solution were respectively added, and the mixture was allowed to react at 37°C for 30 to 60 minutes. Subsequently, the color reaction was analyzed based on the absorbance at 490 nm.
The results are shown in Table 2. The values of the dopa oxidase activity in Table 2 are indicated as values relative to the fluorescence intensities of the instances where sample were not added.

**[Table 2]**

| Compound | Concentration | Dopa oxidase activity (%) | Remark |
|---|---|---|---|
| Toosendanin | 100nM | 15 | Present invention |
| Kojic acid | 10µM | No inhibitory activity | Reference example |
| Kojic acid | 1mM | 55 | Reference example |

As it is obvious from the results of Table 2, the dopa oxidase activity was decreased to a large extent in the system incorporated with toosendanin isolated from *Melia toosendan* Sieb. et Zucc., thus, it is understood that the dopa oxidase activity can be effectively inhibited by toosendanin. Particularly, the system could inhibit the dopa oxidase activity even at a very low concentration as compared to kojic acid, which is a known skin-lightening component. Furthermore, as described above, the dopa oxidase activity is used as an index for the enzyme activity of tyrosinases which is involved in melanin biosynthesis. Therefore, it is understood that toosendanin can suppress melanin production by inhibiting the dopa oxidase activity, and is useful as a skin-lightening component.

### (Prescription Example)

A lotion, an emulsion, a serum, a cream and a facial pack having the compositions shown below were respectively prepared by conventional methods, using an extract obtained in the Preparation Example as an active ingredient.

**1. Preparation of lotion**

| (Component) | (Content: mass%) |
|---|---|
| Toosendanin | 0.1 |
| 1,3-Butylene glycol | 8.0 |
| Glycerol | 5.0 |
| Ethanol | 3.0 |
| Chamomile extract | 3.0 |
| Bellflower extract | 1.0 |
| Clove extract | 1.0 |
| Xanthane gum | 0.1 |
| Hyaluronic acid | 0.1 |
| Disodium hydrogen phosphate | 0.1 |
| Sodium dihydrogen phosphate | 0.1 |
| Glucoside 2-ascorbate | 2.0 |
| Purified water | Balance |
| Perfume | Moderate amounts |
| Preservative | Moderate amounts |

**2. Preparation of emulsion**

| (Component) | (Content: mass%) |
|---|---|
| Toosendanin | 0.5 |
| Chamomile extract | 1.0 |
| Bellflower extract | 1.0 |
| Althea extract | 2.0 |
| Squalane | 3.0 |
| Oil of olive | 3.0 |
| Glycerol | 5.0 |
| Polyoxyethylene hydrogenated castor oil (Number of added moles of ethylene oxide: 40) | 1.0 |
| Carboxyvinyl polymer | 0.2 |
| Potassium hydroxide | 0.1 |
| Xanthane gum | 0.2 |
| Disodium edetate | 0.02 |
| Purified water | Balance |
| Preservative | Moderate amounts |

**3. Preparation of serum**

| (Component) | (Content: mass%) |
|---|---|
| Toosendanin | 0.5 |
| Chamomile extract | 1.0 |
| Bellflower extract | 1.0 |
| Clove extract | 1.0 |
| Carboxyvinyl polymer | 0.2 |
| Acrylate-methacrylate alkyl copolymer | 0.2 |
| Potassium hydroxide | 0.2 |
| Xanthane gum | 0.1 |
| Hyaluronic acid | 0.2 |
| Sodium citrate | 0.15 |
| Citric acid | 0.03 |
| Glycerol | 10.0 |
| 1,3-Butylene glycol | 5.0 |
| Disodium edetate | 0.05 |
| Purified water | Balance |
| Preservative | Moderate amounts |
| Perfume | Moderate amounts |

**4. Preparation of cream**

| (Component) | (Content: mass%) |
|---|---|
| Toosendanin | 1.0 |
| Chamomile extract | 2.0 |
| Bellflower extract | 2.0 |
| Clove extract | 2.0 |
| Methylpolysiloxane | 3.0 |
| Squalane | 2.0 |
| Neopentyl glycol dicaprate | 3.0 |
| Stearyl alcohol | 1.5 |
| Cetanol | 1.0 |
| Polyoxyethylene hydrogenated castor oil (Number of added moles of ethylene oxide: 60) | 0.5 |
| Acrylate-methacrylate alkyl copolymer | 0.3 |
| Potassium hydroxide | 0.15 |
| Xanthane gum | 0.1 |
| Disodium edetate | 0.05 |
| Purified water | Balance |
| Preservative | Moderate amounts |
| Perfume | Moderate amounts |

**5. Preparation of facial pack**

| (Component) | (Content: mass%) |
|---|---|
| Toosendanin | 1.0 |
| Chamomile extract | 2.0 |
| Bellflower extract | 1.0 |
| Dipropylene glycol | 3.0 |
| Polyethylene glycol (number average molecular weight 1500) | 2.0 |
| 1,3-Butylene glycol | 5.0 |
| Glycerol | 5.0 |
| Sodium citrate | 0.5 |
| Polyvinyl alcohol | 10.0 |
| lactic acid | 0.3 |
| Polyoxyethylene decyl tetradecyl ether | 0.5 |
| Purified water | Balance |
| Preservative | Moderate amounts |
| Perfume | Moderate amounts |

### INDUSTRIAL APPLICABILITY

The inhibitor of dopa oxidase activity and the skin-lightening agent of the present invention contain, as an active ingredient, a compound having an excellent activity to suppress dopa oxidase activity, and can suppress melanin production. Therefore, the inhibitor of dopa oxidase activity and the skin-lightening agent of the present invention can be used in the field of cosmetics and beauty, and the field of medicament and medical care.

Having described our invention as related to the present embodiments, it is our intention that the invention not be limited by any of the details of the description, unless otherwise specified, but rather be construed broadly within its spirit and scope as set out in the accompanying claims.

This application claims priority on Patent Application No. 2009-263588 filed in Japan on November 19, 2009, which is entirely herein incorporated by reference.

## Claims

1. An inhibitor of dopa oxidase activity, comprising a compound represented by the following Formula (1) as an active ingredient: 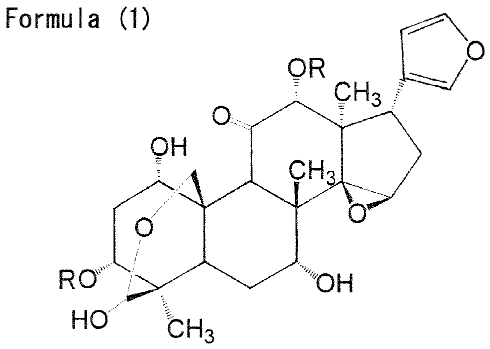 wherein R represents an acyl group having 1 to 5 carbon atom(s).

2. A skin-lightening agent, comprising a compound represented by the following Formula (1) as an active ingredient: 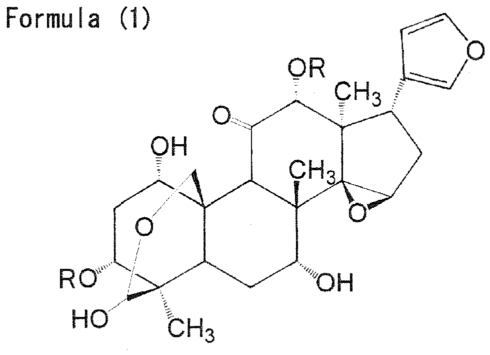 wherein R represents an acyl group having 1 to 5 carbon atom(s).

3. A compound represented by the following Formula (1) for use in the suppression of dopa oxidase activity: 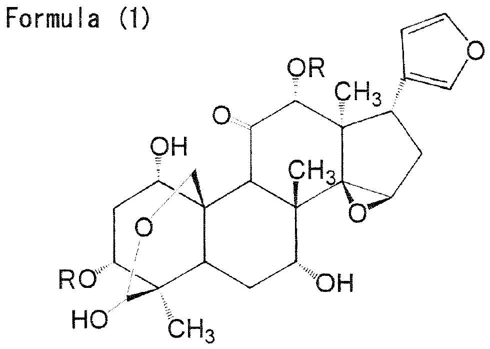 wherein R represents an acyl group having 1 to 5 carbon atom(s).

4. A compound represented by the following Formula (1) for use in the skin-lightening: 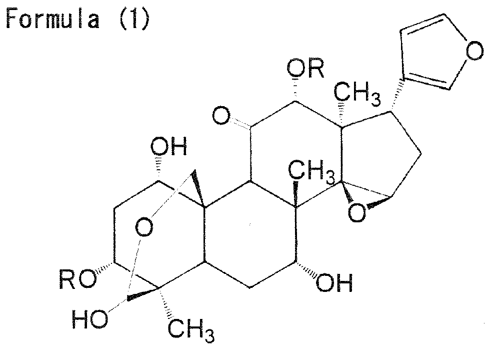 wherein R represents an acyl group having 1 to 5 carbon atom(s).

5. Use of a compound represented by the following Formula (1) for the preparation of a medicament having an effect of suppressing dopa oxidase activity: 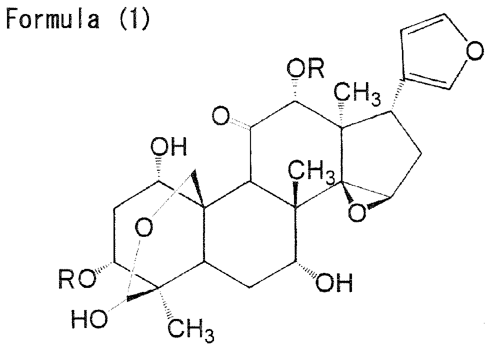 wherein R represents an acyl group having 1 to 5 carbon atom(s).

6. Use of a compound represented by the following Formula (1) for the preparation of a medicament having an effect of skin-lightening: 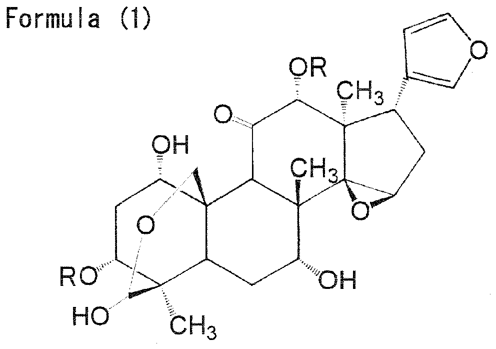 wherein R represents an acyl group having 1 to 5 carbon atom(s).
